# FASCICULE DE BREVET EUROPEEN

(11) **EP 1 347 938 B1**
(45) Date de publication et mention de la délivrance du brevet: **07.03.2012**
(21) Numéro de dépôt: 01995764.6
(22) Date de dépôt: 21.12.2001
(51) Int. Cl.: C01B 21/092, C07C 303/02, H01M 10/50, C01B 21/093, C01B 21/097

(54) **PROCEDE DE FLUORATION D'UN COMPOSE COMPRENANT UN GROUPE HALOSULFONYLE OU DIHALOPHOSPHONYLE**
VERFAHREN ZUR FLUORIERUNG EINER VERBINDUNG MIT EINER HALOGENSULFONYL- ODER DIHALOGENPHOSPHONYLGRUPPE
METHOD FOR FLUORINATING A COMPOUND COMPRISING A HALOSULPHONYL OR DIHALOPHOSPHONYL GROUP

(30) Priorité: 29.12.2000 FR 0017307
(43) Date de publication de la demande: 01.10.2003
(73) Titulaire: HYDRO-QUEBEC, Montréal, Québec H2X 3P3 (CA)
(72) Inventeur: CERNIK, Milos, 62100 Brno (CZ); RUZICKA, Antonin, 67903 Olomucany (CZ); ZAK, Zdirad, 62900 Brno (CZ); PEVERE, Virginie, F-69008 Lyon (FR); MICHOT, Christophe, F-38000 Grenoble (FR)
(74) Mandataire: Sueur, Yvette
(86) Numéro de dépôt international: PCT/FR2001/004164
(87) Numéro de publication internationale: WO 2002/053494

(56) Documents cités:
- US-A- 3 920 738
- RUFF ET AL.: "Imidodisulfuryl fluoride, cesium imidodisulfuryl fluoride and fluoroimidodisulfuryl fluoride" INORGANIC SYNTHESES, vol. 11, 1968, pages 138-43, XP001027947 cité dans la demande
- APPEL ET AL.: "Die synthese des Imidobisschwefelsäurefluorids, HN(SO2F)2" CHEM. BER., vol. 95, 1962, pages 246-8, XP002179138 cité dans la demande
- BARBOUR A K ET AL: "THE PREPARATION OF ORGANIC FLUORINE COMPOUNDS BY HALOGEN EXCHANGE" ADVANCES IN FLUORINE CHEMISTRY, XX, XX, vol. 3, 1963, pages 181-250, XP001027931 cité dans la demande
- SARTORI ET AL.: "Elektrochemische Perfluorierung mit Mehrkomponenten-Elektrolyten" DECHEMA MONOGRAPHIEN: ELECTROCHEMISCHE STOFFGEWINNUNG-GRUNDLAGEN UND VERFAHRENSTECHNIK, vol. 125, 1992, pages 233-242, XP001028663

## Description

La présente invention concerne un procédé de fluoration pour l'obtention de composés fluorés utilisables notamment comme électrolyte.

Les batteries au lithium dans lesquelles l'anode est constituée par une feuille de lithium métallique ou par un alliage de lithium, et qui fonctionnent par circulation d'ions lithium entre les électrodes, ont été largement étudiées. Leur développement a cependant été freiné par le fait que, lors de leur recharge, il se produit un dépôt de lithium métallique de nature dendritique qui peut induire des courts-circuits conduisant à une explosion du système. Ce risque a été supprimé en remplaçant l'anode de lithium ou d'alliage de lithium par une anode constituée par un matériau carboné dans lequel les ions lithium peuvent être insérés de manière réversible. Cette nouvelle forme de batteries au lithium, dites batteries "lithium-ion", est largement utilisé dans le domaine de l'électronique portable. L'électrolyte de ces batteries comprend au moins un sel de lithium en solution dans un solvant organique qui peut être un solvant liquide polaire aprotique (par exemple le carbonate d'éthylène, le carbonate de propylène, un carbonate de dialkyle) éventuellement supporté par un support plastique poreux, un polymère polaire [par exemple un poly(oxyde d'éthylène) réticulé] ou un solvant liquide gélifié par un polymère. Le sel de lithium joue un rôle important dans le fonctionnement de la batterie. Le sel le plus largement utilisé est LiPF₆ qui permet d'obtenir des électrolytes liquides qui ont une conductivité supérieure à 10⁻² S.cm⁻¹ à température ambiante. Il présente cependant une stabilité thermique limitée, ce qui provoque la formation de LiF et de HF, ledit HF induisant une décomposition de l'électrolyte qui peut conduire à une explosion de la batterie. Le sel de lithium du bis(trifluorométhanesulfonyl)imide a été envisagé pour remplacer LiPF₆, mais il présente l'inconvénient de provoquer la dépassivation du collecteur de courant en aluminium de la cathode.

On a alors étudié l'utilisation d'imidures ou de méthides ayant des groupes électro-attracteurs FSO₂ ou F₂PO (WO95/26056). Ces sels permettent d'obtenir des électrolytes plus conducteurs que leurs homologues comprenant des groupes perfluoroalkyles à la place des atomes de fluor et ils produisent une corrosion nettement plus faible des collecteurs en aluminium. L'utilisation d'un imidure ou d'un méthide comprenant des groupes FSO₂ ou F₂PO permet ainsi de maintenir le faible niveau de corrosion constaté avec LiPF₆ tout en améliorant la stabilité thermique par rapport à celle de LiPF₆.

Divers procédés pour la préparation d'imidures ou de méthides comprenant au moins un groupe FSO₂ ou F₂PO ont été décrits. Par exemple, le bis(fluorosulfonyl)imide (FSO₂)₂NH peut être préparé par réaction de l'acide fluorosulfonique FSO₃H avec l'urée H₂NC(O)NH₂. L'imide est ensuite isolé par traitement du mélange réactionnel par NaCl dans le dichlorométhane, suivi d'une distillation de l'acide pur. [Appel & Eisenhauer, Chem. Ber. 95, 246-8, 1962]. La toxicité et le caractère corrosif de FSO₃H constituent cependant un inconvénient majeur.

Un autre procédé consiste à faire réagir (ClSO₂)₂NH avec AsF₃. L'acide (FSO₂)₂NH est ensuite isolé en traitant le mélange réactionnel par NaCl dans le dichlorométhane [Ruff et Lustig, Inorg. Synth 1968, 11, 138-43]. L'inconvénient de ce procédé réside notamment dans le coût élevé de AsF₃, dans sa toxicité et dans le risque de polluer le composé obtenu.

Pour les dérivés phosphoryles, un procédé pour la préparation de LiN(POF₂)₂ consiste à faire réagir LiN(SiMe₃)₂ avec POF₃. L'élimination de Me₃SiF volatil conduit directement au produit attendu [Fluck et Beuerle, Z. Anorg. Allg. Chem. 412(1), 65-70, 1975]. L'inconvénient de ce procédé réside dans le coût du dérivé silylé et l'utilisation de POF₃ gazeux et toxique.

Il est connu de préparer un composé fluoré à partir du composé halogéné correspondant par une réaction d'échange d'halogène à l'aide d'un halogénure ionique, tel que par exemple KF, CsF, ou un fluorure organique tel que le fluorure de tétra-n-butylammonium. La réaction est une substitution nucléophile qui a lieu de préférence dans un solvant polaire aprotique. La réaction d'échange est favorisée par la présence d'un catalyseur de transfert de phase, choisi par exemple parmi les sels d'ammonium quaternaire, les éthers couronnes, les sels de pyridinium, les sels de phosphonium quaternaires. Ce procédé a été mis en oeuvre avec KF notamment pour obtenir des monofluoroalcanes, des α-fluoroesters, des fluoroéthers, des fluorures d'acyles, des fluorures de sulfonyles, respectivement à partir des monohaloalcanes, des α-haloesters, des haloéthers, des halogénures d'acyle, ou des halogénures de sulfonyle correspondants. [A. Basbour, et al, in M. Stacy and co-eds, Advances in Fluorine Chemistry, Vol. 3, Butterworks, Washington, D.C. 1963, pp. 181-250].

Les inventeurs ont maintenant trouvé que, de manière surprenante, le procédé d'échange halogène fluor pouvait être mis en oeuvre pour la fluoration de divers composés comprenant au moins un groupe halosulfonyle ou dihalophosphoryle attaché à un atome portant au moins un substituant fortement électro-attracteur et éventuellement un hydrogène acide.

Le but de la présente invention est par conséquent de fournir un procédé de fluoration d'un composé comprenant au moins un groupe halosulfonyle ou dihalophosphoryle dans lequel l'halogène est différent d'un fluor et au moins un groupe fortement électro-attracteur, en vue notamment de la préparation des composés correspondants comprenant au moins un groupe fluorosulfonyle ou difluorophosphoryle.

Le procédé de fluoration selon la présente invention consiste à faire réagir, éventuellement dans un solvant, un agent fluorant avec un composé (I) comprenant un substituant halosulfonyle dans lequel l'halogène est différent d'un fluor. Il est caractérisé en ce que l'agent fluorant est un fluorure ionique d'un cation monovalent et en ce que le composé (I) répond à formule suivante : 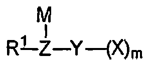 dans laquelle :
- M représente H, un métal alcalin, un groupe phosphonium quaternaire ou un groupe ammonium quaternaire ;
- Z représente CR², N ou P ;
- Y représente SO₂ et m est 1, ou bien Y est PO et m est 2 ;
- R¹ représente un groupe électro-attracteur qui a un paramètre σ_{P} de Hammett supérieur à 0,4 ;
- R² représente un groupe carboné et/ou électro-attracteur ;
- X représente un halogène différent d'un fluor.

Le procédé est particulièrement préféré pour les composés dans lesquels Z représente N.

Le procédé est avantageusement mis en oeuvre à la pression atmosphérique, à une température inférieure à 180°C. De préférence, la température est inférieure à 100°C, plus particulièrement inférieure à 80°C. La mise en oeuvre à une température inférieure à la température ambiante a pour effet une vitesse de réaction trop lente. Le chauffage du milieu réactionnel peut être effectué par les moyens conventionnels. On peut également chauffer à l'aide de micro-ondes. L'agitation du milieu réactionnel ou l'application d'ultrasons est utile pour renouveler la surface active des réactifs lorsqu'ils sont en suspension.

Le fluorure ionique monovalent peut être un fluorure alcalin ou un fluorure d'un cation onium stable. Parmi les métaux alcalins, il est avantageux d'utiliser KF ou CsF. Parmi les cations onium, les cations tétraalkylammonium, tétraalkylphosphonium ou dialkylsulfonium sont préférés. Les cations onium dans lesquels les radicaux alkyles (qui peuvent être identiques ou différents dans un cation onium) ont de 1 à 12, plus particulièrement de 1 à 4 atomes de carbone sont préférés. Les fluorures d'onium précités sont intéressants en raison de leur grande solubilité dans les solvants organiques. Ils peuvent donc être utilisés seuls, ou en association avec un fluorure ionique moins soluble pour lequel ils jouent alors le rôle de catalyseur de transfert de charge. Lorsque le cation M du composé (I) est un métal alcalin ou un onium tel que défini ci-dessus pour le fluorure, il est avantageux d'utiliser un fluorure dudit cation M. L'utilisation de LiF ou de NaF, bien que donnant des réactions relativement lentes, est intéressante lorsque le produit fluoré obtenu à partir du composé (I) est destiné à être utilisé comme électrolyte. Il est préférable d'utiliser un fluorure ionique ayant une surface active élevée.

La quantité de fluorure ionique utilisée par rapport à la quantité de composé (I) est de préférence supérieure à la stoechiométrie. Le rapport du nombre de moles fluorure au nombre d'atomes d'halogène à échanger du composé (I) est avantageusement de 1,1 à 2. Lorsque le composé (I) est un imide [M est H dans la formule (I)], ledit rapport est de préférence supérieur à 2, plus particulièrement supérieur à 3.

Le procédé de la présente invention est particulièrement adapté pour la fluoration de composés (I) dans lesquels M est H ou un métal alcalin, choisi par exemple parmi Na, K, Li ou Cs. Lorsque M est un ammonium quaternaire ou un phosphonium quaternaire, il répond respectivement aux formules N(R³R⁴R⁵R⁶) et P(R³R⁴R⁵R⁶) dans lesquelles les différents substituants Rⁱ sont choisis indépendamment les uns des autres parmi les radicaux alkyles ayant de préférence de 1 à 12, plus particulièrement de 1 à 4 atomes de carbone.

R¹ est un radical électro-attracteur ayant un paramètre σ_{P} de Hammett supérieur à 0,4. Les radicaux ayant un σ_{P} supérieur à 0,5, plus particulièrement supérieur à 0,7 sont particulièrement préférés. De préférence, le radical R¹ ne porte pas de charge positive à moins de 6 chaînons de Z. Comme exemples de radicaux R¹ on peut citer :
- les radicaux X' SO₂- et (X')₂PO- dans lesquels le groupe X' représente ou les deux groupes X' représentent indépendamment l'un de l'autre :
   - un halogène,
   - un radical R⁷CF₂- dans lequel R⁷ est un halogène différent de F ou un radical carboné ayant de préférence au plus 15 atomes de carbone ;
   - un radical perhalogéné R_{F}, ayant de préférence un nombre d'atomes de carbone inférieur ou égal à 15, répondant à la formule R^{B} (CX"₂)ₚ- dans laquelle :
      * chacun des X" représente indépendamment l'un de l'autre F, Cl ou un radical perfluoroalkyle ayant de 1 à 5 atomes de carbone (de préférence 2 atomes de carbone), au moins l'un des X" étant F, de préférence porté par le carbone relié au soufre, p étant 1 ou 2 ;
      * R⁸ est un atome ou un radical électro-attracteur ayant un σₚ supérieur à 0, (de préférence supérieur à 0,1, plus particulièrement supérieur à 0,2) dont les éventuelles fonctions sont inertes dans les conditions de réaction, par exemple un F ou un perfluoroalkyle ayant au plus 8 atomes de carbone ;
- divers radicaux ayant un σₚ supérieur à 0,4, cités notamment dans Advanced Organic Chemistry, 3ème Ed. Gerry March, p. 244, tels que par exemple COOR', COR', SO₂R', PO(R')₂ ou PO(OR')₂ dans lesquels R' est de préférence un radical alkyle ayant de 1 à 15 atomes de carbone ou un radical aryle ayant de 6 à 20 atomes de carbone.

Dans un mode de réalisation préféré, R¹- représente un radical X'SO₂- ou (X')₂PO- tel que défini ci-dessus.

Le substituant R² représente un radical carboné et/ou électro-attracteur. Lorsque R² est un radical électro-attracteur, il est choisi avantageusement parmi le radical nitrile et les radicaux définis ci-dessus pour R¹. Lorsque R² est un groupe carboné, il est choisi de préférence parmi les radicaux ayant de 1 à 20 atomes de carbone.

Lorsque le composé (I) est liquide à la température de la réaction et que le fluorure ionique est soluble dans ledit composé liquide, il n'est pas indispensable d'ajouter un solvant au milieu réactionnel.

Lorsque les deux réactifs sont sous forme solide, la réaction est effectuée dans un solvant liquide. Le solvant est aprotique lorsque M est différent de H.

Lorsque la solvatation du cation du réactif fluorure monovalent est recherchée, on utilise de préférence un solvant ayant un nombre donneur de 10 à 30, de préférence de 20 à 30. L'indice donneur d'un solvant représente la valeur -ΔH, ΔH étant l'enthalpie (en Kcal/mol) de l'interaction entre le solvant et le pentachlorure d'antimoine dans une solution diluée de dichlorométhane [Cf. Christian Reinhardt, Solvant and solvant effects in Organic Chemistry WCH, p. 19, 1988].

Les solvants donnant de bons résultats peuvent être notamment des amides, y compris les amides à caractère particulier tels que les urées tétrasubstituées et les lactames monosubstitués. Les amides sont, de préférence, substitués (disubstitués pour les amides ordinaires). On peut citer par exemple les dérivés de pyrrolidone tels que la N-méthylpyrrolidone, le N,N-diméthylformamide, ou le N,N-diméthylacétamide. Une autre catégorie particulièrement intéressante de solvants est constituée par les éthers, symétriques ou non symétriques, ouverts ou non, y compris les différents dérivés des éthers de glycol tels que les glymes, par exemple le diglyme. Ainsi les solvants les plus adéquats, en raison de leur prix et de leurs propriétés, sont avantageusement choisis parmi les éthers (notamment les éthers cycliques tels que le THF ou les éthers polyfonctionnels tels les glymes), ou parmi les amides qui n'ont pas d'hydrogène acide, tels que le DMF ou les N,N'-Dialkylalkylèneurées parmi lesquelles on peut citer la DMEU (N,N'-DiMéthylEthylène-Urée) ou la DMPU (N,N'-DiMéthylPropylèneUrée). On peut également citer la N-méthylpyrolidone et les urées cycliques peralkylées sur les azotes (e.g. DMEU ou DMPU).

Le solvant peut en outre être le nitrométhane.

Il peut être intéressant d'ajouter au milieu réactionnel un catalyseur de transfert de phase, afin d'améliorer le rendement de la réaction. Cet ajout est particulièrement utile lorsque la réaction est effectuée dans un solvant non polaire ou peu polaire. Comme exemple de catalyseur de transfert de phase, on peut citer les sels d'ammonium quaternaire, les éthers couronnes, les sels de pyridinium, les sels de phosphonium quaternaires. L'addition d'un catalyseur de transfert de phase vise à remédier à une solubilité relativement faible du fluorure ionique alcalin utilisé. Un fluorure ionique fortement soluble utilisable comme réactif de fluoration dans le procédé de la présente invention peut être utilisé comme catalyseur de transfert de phase lorsqu'il est associé à un réactif fluorure à faible solubilité. A titre d'exemple, on peut citer les fluorures d'onium et le fluorure de césium.

Les composés (I) peuvent être préparés par des procédés de l'art antérieur. Un imidure peut être préparé par action sur l'imide correspondant d'un sel dont la forme acide est volatile dans les conditions de la réaction. Par exemple, l'action d'un hydrure alcalin sur un imide en milieu protique permet d'obtenir un imidure anhydre. On peut également faire réagir un composé alcoyl-métal, par exemple le butyllithium, sur un imide, pour obtenir l'imidure correspondant et un alcane qui est volatil s'il s'agit d'un alcane inférieur. On peut en outre obtenir un imidure à partir de l'imide correspondant par échange avec un carboxylate de poids moléculaire suffisamment faible pour que l'acide carboxylique correspondant soit volatil.

La présente invention est illustrée par les exemples suivants, auxquels elle n'est cependant pas limitée.

### Exemple 1

On a introduit dans un réacteur 3,556 g (137,1 mM) de LiF dans 5 ml de nitrométhane, puis on a agité pendant 18 h en présence de billes de verre. On a ensuite ajouté goutte à goutte sous agitation une solution de 4,907 g (0,22926 mM) de bis(chlorosulfonyl)imide dans 5 ml de nitrométhane, correspondant à un rapport molaire LiF/imide d'environ 6. On a laissé la réaction se poursuivre durant la nuit. Un résidu solide a décanté et la solution surnageante a été récupérée pour analyse par RMN du fluor.

L'analyse a montré la présence de singulets du fluor à divers déplacements chimiques et avec des hauteurs de pics différentes :

| Déplacement chimique | hauteur de pic | espèce |
|---|---|---|
| 56,6 | 1 | FSO₂NH₂ |
| 50,8 | 78,4 | (SO₂F) (SO₂Cl) NH |
| 54,5 | 12,7 | SO₂F |
| 35,3 | 53,3 | FSO₃⁻ |

La réaction s'étant poursuivie pendant deux semaines, on a obtenu des hauteurs de pic différentes, le composé majoritaire étant le bis(fluorosulfonyl)imidure de lithium souhaité.

### Exemple 2

On a dissous 4,111 g (19,205 mM) de bis(chlorosulfonyl)-imide dans 5 ml de nitrométhane. Sous agitation continue, on a ajouté 6,549 g (155,97 mM) de NaF finement divisé, à 0°C. Le rapport molaire NaF/imide est de l'ordre de 8. On a laissé le mélange réactionnel monter à température ambiante, puis on a agité en présence de 3 billes de verre pendant 60 h. Après décantation, l'analyse RMN du fluor de la solution claire surnageante a montré la présence du produit désiré.

### Exemple 3

On a mis 4,361 g (75,06 mM) de KF en suspension dans 5 ml de nitrométhane, puis on a introduit sous agitation continue, une solution de 3,176 g (14,84 mM) de bis(chlorosulfonyl)imide dans 3 ml de nitrométhane. Le rapport molaire KF/imide est de l'ordre de 5.

Le mélange réactionnel s'est échauffé et le réacteur a été agité avec des billes de verre pendant 14 h. On a alors ajouté 3,49 g (60,034 mM) de KF frais et on a agité à nouveau pendant 18 h. La solution est devenue orange profond. Après décantation des particules solides, on a fait l'analyse RMN du fluor qui a montré que le produit majoritaire présente un déplacement chimique (singulet) de 51,6 ppm et correspond à la transformation de 99% du produit initial. Le produit majoritaire obtenu est le bis(fluorosulfonyl)imidure de potassium.

On a mis en oeuvre le mode opératoire de cet exemple dans trois essais supplémentaires, en utilisant à la place du bis (chlorosulfonyl) imide, respectivement φSO₂NHSO₂Cl, CF₃SO₂NHSO₂Cl et (φSO₂)₂CHSO₂Cl, et l'on a observé la formation majoritaire des composés suivants : φSO₂NKSO₂F (à partir de φSO₂NHSO₂Cl), CF₃SO₂NKSO₂F (à partir de CF₃SO₂NHSO₂Cl) et (φSO₂)₂CKSO₂F à partir de (φSO₂)₂CHSO₂Cl.

### Exemple 4

On a dispersé 4,421 g (29,104 mM) de CsF dans 2 ml de nitrométhane en agitant avec des billes de verre. Sous agitation, on a ajouté goutte à goutte une solution de 2,243 g (10,480 mM) de bis(chlorosulfonyl)imide dans 5 ml de nitrométhane. Le rapport molaire CsF/imide est de l'ordre de 3. Après une durée de réaction de 72 h suivie d'une agitation pendant 6 h, l'analyse RMN du fluor du liquide surnageant a montré les raies suivantes :

| Déplacement chimique | hauteur de pic | espèce |
|---|---|---|
| 56,5 | 15,5 | FSO₂NH₂ |
| 52,1 | 48,3 | (SO₂F) (SO₂Cl) N⁻ |
| 51,9 | 225, 6 | [N(SO₂F)²]⁻ |

Il apparaît ainsi que 80% de l'imide de départ a été converti en bis(fluorosulfonyl)imide.

### Exemple 5

Par un procédé analogue à celui décrit dans l'exemple 3, on a fait réagir la bis(dichlorophosphoryl)imide avec KF. On a constaté que la transformation du produit de départ avec une rendement de 90% et la formation majoritaire de bis(difluorophosphoryl)imide de potassium.

La bis(dichlorophosphoryl)imide peut être préparée suivant le procédé décrit par Riesel et al., [Riesel, Pfuetzner & Herrmann, Z. Chem. 23(9), 344-5, 1983].

## Revendications

1. Procédé de fluoration d'un composé (I) comprenant au moins un groupe halosulfonyle ou dihalophosphoryle dans lequel l'halogène est différent d'un fluor et au moins un groupe fortement électro-attracteur, consistant à faire réagir, éventuellement dans un solvant, un agent fluorant avec ledit composé, **caractérisé en ce que** l'agent fluorant est un fluorure ionique d'un cation monovalent et **en ce que** le composé (I) répond à formule suivante : 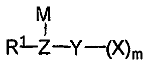 dans laquelle :
• M représente H, un métal alcalin, un groupe phosphonium quaternaire ou un groupe ammonium quaternaire ;
• Z représente CR², N ou P ;
• Y représente SO₂ et m est 1, ou bien Y est PO et m est 2 ;
• R¹ représente un groupe électro-attracteur qui a un paramètre σₚ de Hammett supérieur à 0,4 ;
• R² représente un groupe carboné et/ou électro-attracteur ;
• X représente un halogène différent d'un fluor.

2. Procédé selon la revendication 1, **caractérisé en ce qu'**il est mis en oeuvre à la pression atmosphérique

3. Procédé selon la revendication 1, **caractérisé en ce qu'**il est mis en oeuvre à une température inférieure à 180°C.

4. Procédé selon la revendication 1, **caractérisé en ce que** le fluorure ionique monovalent est KF ou CsF.

5. Procédé selon la revendication 1, **caractérisé en ce que** le fluorure ionique monovalent est un fluorure de tétraalkylammonium, de tétraalkylphosphonium ou de dialkylsulfonium.

6. Procédé selon la revendication 5, **caractérisé en ce que** les groupes alkyles du cation du fluorure monovalent ont de 1 à 12 atomes de carbone.

7. Procédé selon la revendication 1, **caractérisé en ce que** le rapport du nombre de moles fluorure au nombre d'atomes d'halogène à échanger du composé (I) est supérieur à 1.

8. Procédé selon la revendication 7, **caractérisé en ce que** le rapport du nombre de moles fluorure au nombre d'atomes d'halogène à échanger du composé (I) est de 1,1 à 2.

9. Procédé selon la revendication 7, **caractérisé en ce que** le rapport du nombre de moles fluorure au nombre d'atomes d'halogène à échanger du composé (I) est supérieur à 2 lorsque M est H.

10. Procédé selon la revendication 1, **caractérisé en ce que** M représente H, un métal alcalin, un ammonium quaternaire N (R³R⁴R⁵R⁶) ou un phosphonium quaternaire P (R³R⁴R⁵R⁶) , les différents substituants Rⁱ étant choisis indépendamment les uns des autres parmi les radicaux alkyles ayant de préférence de 1 à 12 atomes de carbone.

11. Procédé selon la revendication 1, **caractérisé en ce que** le cation M est identique au cation du fluorure monovalent.

12. Procédé selon la revendication 1, **caractérisé en ce que** R¹ est un groupe électro-attracteur ayant un paramètre σₚ de Hammett supérieur à 0,7.

13. Procédé selon la revendication 1, **caractérisé en ce que** R¹ et/ou R² sont un radical X'SO₂- ou (X')₂PO- dans lequel X' représente :
- un halogène,
- un radical R⁷CF₂- dans lequel R⁷ est un halogène différent de F ou un radical carboné ;
- un radical perhalogéné R_{F} répondant à la formule R⁸ (CX"₂)ₚ- dans laquelle :
* chacun des X" représente indépendamment l'un de l'autre F, Cl ou un radical perfluoroalkyle ayant de 1 à 5 atomes de carbone, au moins l'un des X" étant F, p étant 1 ou 2 ;
* R⁸ est un atome ou un radical électro-attracteur ayant un σₚ supérieur à 0, dont les éventuelles fonctions sont inertes dans les conditions de réaction.

14. Procédé selon la revendication 13, **caractérisé en ce que** R⁷ est un radical carboné ayant au plus 15 atomes des carbone.

15. Procédé selon la revendication 13, **caractérisé en ce qu'**au moins l'un des X" représente un radical perfluoroalkyle ayant de 1 à 5 atomes de carbone.

16. Procédé selon la revendication 13, **caractérisé en ce qu'**au moins l'un des X" est un atome de F porté par le carbone relié au soufre.

17. Procédé selon la revendication 13, **caractérisé en ce que** R⁸ est F ou un radical perfluoroalkyle ayant au plus 8 atomes de carbone.

18. Procédé selon la revendication 1, **caractérisé en ce que** R¹ représente un radical COOR', COR', SO₂R', PO(R')₂ ou PR(OR')₂ dans lequel R' est un radical alkyle ayant de 1 à 15 atomes de carbone ou un radical aryle ayant de 6 à 20 atomes de carbone.

19. Procédé selon la revendication 1, **caractérisé en ce que** R² est un nitrile ou un radical carboné ayant de 1 à 20 atomes de carbone.

20. Procédé selon la revendication 1, caractérisé en que ce qu'il est mis en oeuvre dans un solvant aprotique.

21. Procédé selon la revendication 1, **caractérisé en ce que** le solvant est le nitrométhane.

22. Procédé selon la revendication 1, **caractérisé en ce qu'**il est mis en oeuvre dans un solvant choisi parmi les amides substitués ou non substitués et les éthers symétriques ou non, cycliques ou non.

23. Procédé selon la revendication 1, **caractérisé en ce que** le milieu réactionnel contient un catalyseur de transfert de phase.

## Claims

1. A process for the fluorination of a compound (I) comprising at least one halosulfonyl or dihalophosphoryl group in which the halogen is other than a fluorine and at least one strongly electron-withdrawing group, which consists in reacting, optionally in a solvent, a fluorinating agent with said compound, wherein the fluorinating agent is an ionic fluoride of a monovalent cation and wherein the compound (I) corresponds to the following formula: 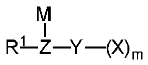 in which:
• M represents H, an alkali metal, a quaternary phosphonium group or a quaternary ammonium group;
• Z represents CR², N or P;
• Y represents SO₂ and m is 1, or else Y is PO and m is 2;
• R¹ represents an electron-withdrawing group which has a Hammett parameter σ_{P} of greater than 0.4;
• R² represents a carbonaceous and/or electron-withdrawing group;
• X represents a halogen other than a fluorine.

2. The process as claimed in claim 1, which is carried out at atmospheric pressure.

3. The process as claimed in claim 1, which is carried out at a temperature of less than 180°C.

4. The process as claimed in claim 1, wherein the monovalent ionic fluoride is KF or CsF.

5. The process as claimed in claim 1, wherein the monovalent ionic fluoride is tetraalkylammonium, tetraalkylphosphonium or dialkylsulfonium fluoride.

6. The process as claimed in claim 5, wherein the alkyl groups of the cation of the monovalent fluoride have from 1 to 12 carbon atoms.

7. The process as claimed in claim 1, wherein the ratio of the number of moles of fluoride to the number of halogen atoms to be exchanged of the compound (I) is greater than 1.

8. The process as claimed in claim 7, wherein the ratio of the number of moles of fluoride to the number of halogen atoms to be exchanged of the compound (I) is from 1.1 to 2.

9. The process as claimed in claim 7, wherein the ratio of the number of moles of fluoride to the number of halogen atoms to be exchanged of the compound (I) is greater than 2 when M is H.

10. The process as claimed in claim 1, wherein M represents H, an alkali metal, a quaternary ammonium N(R³R⁴R⁵R⁶) or a quaternary phosphonium P(R³R⁴R⁵R⁶), the various substituents R¹ being chosen, independently of one another, from alkyl radicals preferably having from 1 to 12 carbon atoms.

11. The process as claimed in claim 1, wherein the cation M is identical to the cation of the monovalent fluoride.

12. The process as claimed in claim 1, wherein R¹ is an electron-withdrawing group having a Hammett parameter σ_{P} of greater than 0.7.

13. The process as claimed in claim 1, wherein R¹ and/or R² are an X'SO₂- or (X')₂PO- radical in which X' represents:
- a halogen,
- an R⁷CF₂- radical in which R⁷ is a halogen other than F or a carbonaceous radical;
- a perhalogenated radical R_{F}, corresponding to the formula R⁸(CX"₂)ₚ- in which:
* each of the X" groups represents, independently of one another, F, Cl or a perfluoroalkyl radical having from 1 to 5 carbon atoms, at least one of the X" groups being F, p being 1 or 2;
* R⁸ is an electron-withdrawing atom or radical having a σₚ of greater than 0, the possible functional groups of which are inert under the reaction conditions.

14. The process as claimed in claim 13, wherein R⁷ is a carbonaceous radical having at most 15 carbon atoms.

15. The process as claimed in claim 13, wherein at least one of the X" groups represents a perfluoroalkyl radical having from 1 to 5 carbon atoms.

16. The process as claimed in claim 13, wherein at least one of the X" groups is a F atom carried by the carbon connected to the sulfur.

17. The process as claimed in claim 13, wherein R⁸ is F or a perfluoroalkyl radical having at most 8 carbon atoms.

18. The process as claimed in claim 1, wherein R¹ represents a COOR', COR', SO₂R', PO(R')₂ or PR(OR')₂ radical in which R' is an alkyl radical having from 1 to 15 carbon atoms or an aryl radical having from 6 to 20 carbon atoms.

19. The process as claimed in claim 1, wherein R² is a nitrile or a carbonaceous radical having from 1 to 20 carbon atoms.

20. The process as claimed in claim 1, which is carried out in an aprotic solvent.

21. The process as claimed in claim 1, wherein the solvent is nitromethane.

22. The process as claimed in claim 1, which is carried out in a solvent chosen from substituted or unsubstituted amides and symmetrical or asymmetrical and cyclic or noncyclic ethers.

23. The process as claimed in claim 1, wherein the reaction medium comprises a phase transfer catalyst.

## Patentansprüche

1. Verfahren zur Fluorierung einer Verbindung (I), die mindestens eine Halogensulfonyl- oder Dihaolgenphosphorylgruppe umfasst, wobei das Halogen von einem Fluor unterschiedlich ist, und mindestens eine elektrisch stark anziehende Gruppe, wodurch bewirkt wird, ein fluorierendes Mittel mit der Verbindung zu einer Reaktion zu veranlassen, eventuell in einem Lösungsmittel, **dadurch gekennzeichnet, dass** das fluorierende Mittel ein ionisches Fluorid eines einwertigen Kations ist und dass die Verbindung (I) der folgenden Formel entspricht: 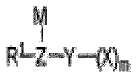 wobei:
• M H, ein Alkalimetall, eine Gruppe quaternäres Phosphonium oder eine Gruppe quaternäres Ammonium darstellt,
• Z CR², N oder P darstellt,
• Y SO₂ darstellt und m 1 ist, oder Y PO ist und m 2 ist,
• R¹ eine elektrisch anziehende Gruppe darstellt, die einen Hammett-Parameter σ_{P} über 0,4 hat,
• R² eine kohlenstoffhaltige und/oder elektrisch anziehende Gruppe darstellt
• X ein Halogen darstellt, das sich von einem Fluor unterscheidet.

2. Verfahren nach Anspruch 1, **dadurch gekennzeichnet, dass** es bei atmosphärischem Druck umgesetzt wird.

3. Verfahren nach Anspruch 1, **dadurch gekennzeichnet, dass** es bei einer Temperatur unter 180 °C umgesetzt wird.

4. Verfahren nach Anspruch 1, **dadurch gekennzeichnet, dass** das einwertige ionische Fluorid KF oder CsF ist.

5. Verfahren nach Anspruch 1, **dadurch gekennzeichnet, dass** das einwertige ionische Fluorid ein Tetraalkylammonium-, Tetraalkylphosphonium- oder Dialkylsulfoniumfluorid ist.

6. Verfahren nach Anspruch 5, **dadurch gekennzeichnet, dass** die Alkylgruppen des Kations des einwertigen Fluorids 1 bis 12 Kohlenstoffatome haben.

7. Verfahren nach Anspruch 1, **dadurch gekennzeichnet, dass** das Verhältnis der Anzahl der Fluoridmole zur Anzahl der auszutauschenden Halogenatome der Verbindung (I) größer als 1 ist.

8. Verfahren nach Anspruch 7, **dadurch gekennzeichnet, dass** das Verhältnis der Anzahl der Fluoridmole zur Anzahl der auszutauschenden Halogenatome der Verbindung (I) 1,2 bis 2 ist.

9. Verfahren nach Anspruch 7, **dadurch gekennzeichnet, dass** das Verhältnis der Anzahl der Fluoridmole zur Anzahl der auszutauschenden Halogenatome der Verbindung (I) größer als 2 ist, wenn M H ist.

10. Verfahren nach Anspruch 1, **dadurch gekennzeichnet, dass** M H, ein Alkalimetall, ein quaternäres Ammonium N (R³R⁴R⁵R⁶) oder ein quaternäres Phosphonium P (R³R⁴R⁵R⁶) darstellt, wobei die verschiedenen Substituenten Rⁱ voneinander unabhängig aus den Alkylradikalen ausgewählt sind, die vorzugsweise 1 bis 12 Kohlenstoffatome haben.

11. Verfahren nach Anspruch 1, **dadurch gekennzeichnet, dass** das Kation M mit dem Kation des einwertigen Fluorids identisch ist.

12. Verfahren nach Anspruch 1, **dadurch gekennzeichnet, dass** R¹ eine elektrisch anziehende Gruppe ist, die einen Hammet-Parameter σₚ über 0,7 hat.

13. Verfahren nach Anspruch 1, **dadurch gekennzeichnet, dass** R¹ und/oder R² ein X'SO₂- oder (X')₂PO-Radikal sind, wobei X' darstellt:
- ein Halogen,
- ein R⁷-CF₂-Radikal, wobei R⁷ ein Halogen ist, das von F unterschiedlich ist oder ein kohlenstoffhaltiges Radikal,
- ein perhalogeniertes Radikal R_{F}, das der Formel R⁸(CX"₂)ₚ- entspricht, wobei:
* jedes der X" unabhängig voneinander F, Cl oder ein Perfluoralkylradikal mit 1 bis 5 Kohlenstoffatomen darstellt, wobei mindestens eines der X" F ist, wobei p 1 oder 2 ist,
* R⁸ ein Atom oder ein elektrisch anziehendes Radikal ist mit einem σₚ über 0, dessen eventuelle Funktionen unter den Reaktionsbedingungen inert sind.

14. Verfahren nach Anspruch 13, **dadurch gekennzeichnet, dass** R⁷ ein kohlenstoffhaltiges Radikal mit höchstens 15 Kohlenstoffatomen ist.

15. Verfahren nach Anspruch 13, **dadurch gekennzeichnet, dass** mindestens eines der X" ein Perfluoralkylradikal mit 1 bis 5 Kohlenstoffatomen darstellt.

16. Verfahren nach Anspruch 13, **dadurch gekennzeichnet, dass** mindestens eines der X" ein Atom von F ist, das von dem Kohlenstoff getragen wird, der mit Schwefel verbunden ist.

17. Verfahren nach Anspruch 13, **dadurch gekennzeichnet, dass** R⁸ F oder ein Perfluoralkylradikal mit höchstens 8 Kohlenstoffatomen ist.

18. Verfahren nach Anspruch 1, **dadurch gekennzeichnet, dass** R¹ ein COOR'-, COR'-, SO₂R'-, PO(R')₂- oder PR(OR')₂-Radikal darstellt, wobei R' ein Alkylradikal mit 1 bis 15 Kohlenstoffatomen oder ein Arylradikal mit 6 bis 20 Kohlenstoffatomen ist.

19. Verfahren nach Anspruch 1, **dadurch gekennzeichnet, dass** R² ein Nitril oder ein kohlenstoffhaltiges Radikal mit 1 bis 20 Kohlenstoffatomen ist.

20. Verfahren nach Anspruch 1, **dadurch gekennzeichnet, dass** es in einem aprotischen Lösungsmittel umgesetzt wird.

21. Verfahren nach Anspruch 1, **dadurch gekennzeichnet, dass** das Lösungsmittel Nitromethan ist.

22. Verfahren nach Anspruch 1, **dadurch gekennzeichnet, dass** es in einem Lösungsmittel umgesetzt wird, das aus den substituierten oder nicht substituierten Amiden und den symmetrischen oder nicht symmetrischen, zyklischen oder nicht zyklischen Ethern ausgewählt ist.

23. Verfahren nach Anspruch 1, **dadurch gekennzeichnet, dass** das Reaktionsmedium einen Phasentransferkatalysator enthält.
